# FASCICULE DE BREVET EUROPEEN

(11) **EP 2 335 756 B1**
(45) Date de publication et mention de la délivrance du brevet: **01.01.2020**
(21) Numéro de dépôt: 10192874.5
(22) Date de dépôt: 29.11.2010
(51) Int. Cl.: A61M 5/48, A61M 5/20, A61M 5/24, A61M 5/32

(54) **INSTRUMENT DE CHIRURGIE POUR L'INJECTION D'UN PRODUIT PHARMACEUTIQUE A TRAVERS UN TISSU DENSE D'UN CORPS HUMAIN**
INSTRUMENT FÜR CHIRURGIE FÜR EIN PHARMAZEUTISCHES INJEKTIONSPRÄPARAT DURCH GEWEBE
SURGICAL INSTRUMENT FOR PHARMACEUTICAL PRODUCT FOR INJECTION INTO THE TISSUE OF A HUMAN BODY

(30) Priorité: 17.12.2009 FR 0959144
(43) Date de publication de la demande: 22.06.2011
(73) Titulaire: Dentalhitec, 49280 Mazieres en Mauges (FR)
(72) Inventeur: Villette, Olivier, 49600 Andreze (FR); Guist'hau, Vincent, 49300 Cholet (FR)
(74) Mandataire: Thinat, Michel

(56) Documents cités:
- EP-A1- 2 022 522
- WO-A1-98/53757
- WO-A1-2009/098666
- US-A1- 2006 106 363

## Description

La présente invention concerne un instrument de chirurgie pour perforer un tissu dense d'un corps humain ou animal et injecter ensuite un produit pharmaceutique dans une zone du corps accessible à travers le tissu dense, selon le préambule de la revendication 1.

Certaines applications médicales, par exemple dans le domaine de l'anesthésie dentaire, nécessitent de traverser des tissus denses, par exemple la corticale osseuse d'une mâchoire, pour pouvoir injecter un produit pharmaceutique au bon endroit. Pour y arriver, on réalise d'abord une perforation avec un foret puis on fait l'injection avec une aiguille. Dans ce cas, il faut réussir à retrouver le trou de perforation avec l'aiguille d'injection. De plus, l'aiguille d'injection étant d'un diamètre plus petit, il y a des risques de fuite de produit pharmaceutique par le trou de perforation pendant l'injection. En outre, cette application en deux étapes demande l'emploi de deux instruments différents, à savoir un perforateur et une seringue, ce qui nécessite plus de temps de réalisation.

Selon un procédé alternatif décrit dans le document EP 2 022 522, on réalise la perforation avec une aiguille perforante servant aussi à l'injection. Ceci permet de gagner du temps et d'éviter des fuites de produit pharmaceutique, puisque le trou de perforation est au diamètre de l'aiguille. Toutefois, cette méthode alternative présente le problème d'une obstruction de la lumière de l'aiguille par les débris générés lors de la perforation. Un procédé du même type est connu de la demande de brevet US 2006/0106363, qui présente donc le même problème que le document EP 2 022 522. De plus, l'instrument par la mise en œuvre du procédé a une structure très complexe.

Le but de l'invention est atteint grâce aux caractéristiques énoncées dans la partie caractérisante de la revendication 1.

D'autres caractéristiques avantageuses de l'invention sont énoncées dans les revendications dépendantes.

Ainsi il est indiqué que l'organe agissant sur le produit pharmaceutique et plus précisément sur un piston intégré dans le contenant, comprend avantageusement sa propre électronique de commande pour pouvoir introduire, pendant une phase de perforation, des petites quantités de produit pharmaceutique dans la lumière de l'aiguille et que le produit pharmaceutique soit maintenu sous pression pendant toute la durée de la perforation. Cette électronique de commande est en outre programmable par exemple de façon que le produit puisse être introduit dans l'aiguille en fonction de l'avancement de la perforation.

Dans la mesure où la lumière de l'aiguille est remplie de produit pharmaceutique sous pression, les débris résultant de la perforation ne peuvent pas y pénétrer et ne peuvent donc pas causer une obstruction de l'aiguille.

L'instrument de l'invention peut avoir l'une au moins des caractéristiques supplémentaires ci-après, considérées isolément ou selon toute combinaison techniquement possible :
- l'instrument comprend un support cylindrique conformé pour recevoir, de manière échangeable, un contenant de produit pharmaceutique, le support cylindrique étant adapté à la fois pour être disposé, dans le logement de la pièce à main, libre en rotation autour d'un axe longitudinal du support cylindrique et pour entraîner en rotation le contenant ;
- le passage axial est pourvu d'un roulement mécanique coaxial servant d'appui pour une extrémité du support cylindrique ;
- la pièce à main comprend un corps allongé comportant le logement pour recevoir le support cylindrique et le contenant, le corps présentant une tête pourvue du passage axial et, à l'extrémité opposée, une pièce de fond amovible comprenant les moyens d'entraînement en rotation du contenant ;
- la tête est un élément distinct monté de manière amovible sur le corps ;
- l'extrémité du support cylindrique en regard du passage axial est pourvue d'une piste de roulement circonférentielle destinée à coopérer avec le roulement mécanique ;
- la piste de roulement circonférentielle est formée par un élément annulaire métallique ou en matière synthétique, selon les charges exercées ;
- le roulement mécanique est un roulement à billes oblique adapté pour pouvoir supporter une charge axiale ;
- l'organe agissant sur le produit pharmaceutique est adapté pour introduire, pendant la perforation d'un tissu dense du corps humain ou animal, dans l'aiguille perforante des quantités de produit pharmaceutique prédéterminées en fonction de l'avancement de la perforation ;
- l'instrument comprend un module de commande électronique relié à l'organe agissant sur le contenant et ayant une mémoire pour stocker des consignes d'injection de quantités prédéterminées du produit pharmaceutique en fonction de l'avancement de la perforation ;
- l'organe agissant sur le produit pharmaceutique est adapté pour entraîner lui-même le contenant et, par l'intermédiaire du support cylindrique, l'aiguille perforante en rotation, ou pour être entraîné par des moyens d'entraînement auxquels il est relié.

Le but de l'invention est également atteint avec un procédé de perforation d'un tissu dense d'un corps humain ou animal et d'injection d'un produit pharmaceutique dans le corps humain ou animal préalablement perforé avec un instrument du type décrit ci avant équipé d'une aiguille perforante.

Conformément à l'invention, le procédé comprend une étape d'introduction et de maintien sous pression d'au moins une quantité prédéterminée du produit pharmaceutique dans l'aiguille au cours de la phase de perforation.

Un tel procédé peut avoir les caractéristiques supplémentaires ci-après, considérées isolément ou selon toute combinaison technique possible :
- le produit pharmaceutique est introduit dans l'aiguille en fonction de l'avancement de la perforation ;
- l'aiguille est maintenue remplie de produit pharmaceutique pendant toute la durée de la perforation pour éviter son obstruction ;
- la phase de perforation est suivie de l'injection d'un produit pharmaceutique de traitement dans le corps humain ou animal.

D'autres caractéristiques et avantages de la présente invention ressortiront de la description ci-après d'un mode de réalisation de l'instrument selon l'invention et de la mise en œuvre d'un procédé selon l'invention. La description est faite en référence aux dessins dans lesquels :
- la figure 1 représente un instrument de chirurgie selon l'invention,
- la figure 2 représente un support cylindrique avec une cartouche en tant que contenant d'un produit pharmaceutique en état d'assemblage,
- la figure 3 représente le support cylindrique et la cartouche séparée,
- la figure 4 représente une vue intérieure de la tête de l'instrument chirurgical,
- la figure 5 représente l'extrémité du support cylindrique destiné à être logé dans la tête de l'instrument de chirurgie, et
- la figure 6 représente le support cylindrique en appui sur le roulement mécanique de la tête de l'instrument de chirurgie.

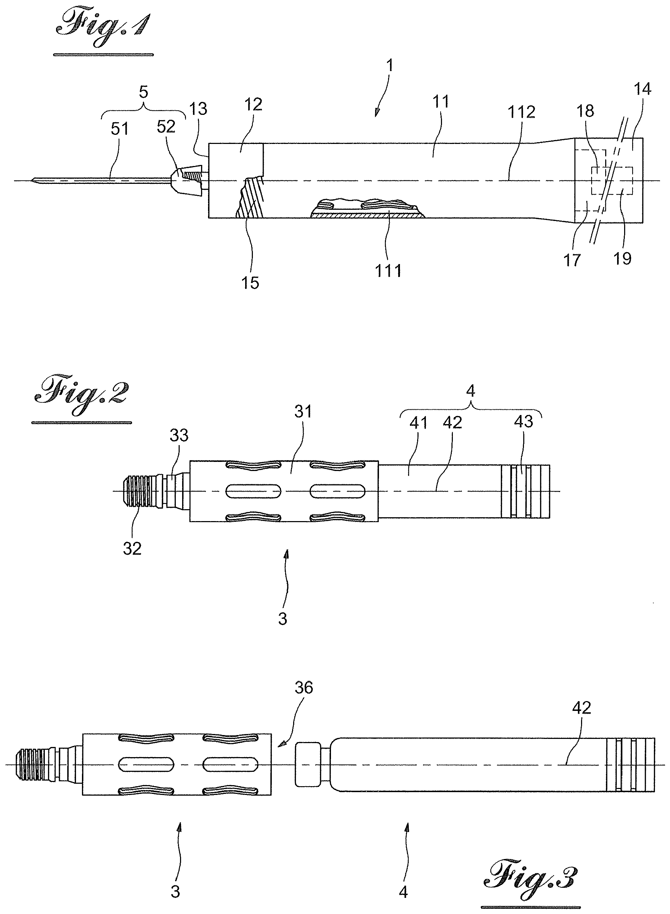

La figure 1 représente, en une vue schématique latérale avec un arrachement, un instrument de chirurgie selon l'invention.

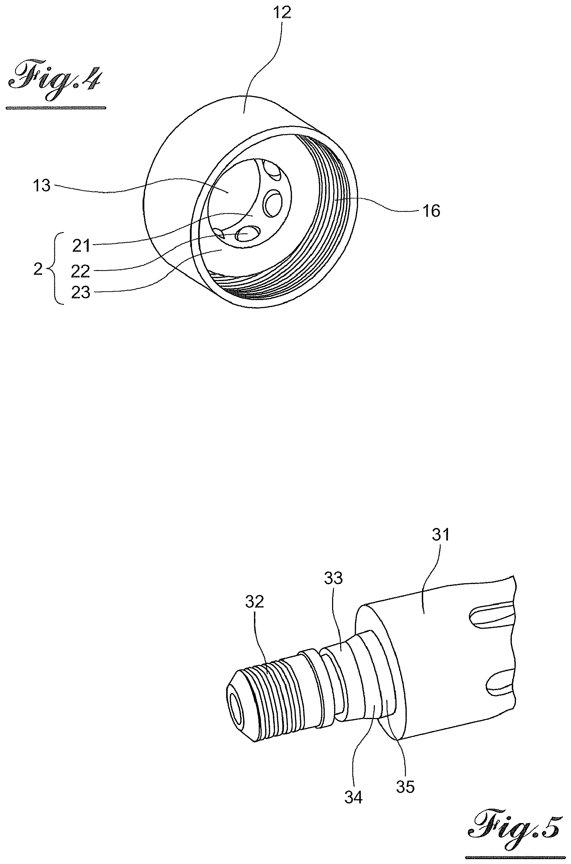

L'instrument comprend une pièce à main 1 ayant un corps allongé 11 avec un logement 111 et deux extrémités opposées 12, 14. L'extrémité 12 est formée par une tête amovible, pourvue d'un passage axial 13. La tête 12 est avantageusement conformée pour être fixée sur le corps 11 par vissage, le corps 11 étant pourvu à cet effet d'un filetage 15 et la tête 12 d'un filetage 16 (voir figure 4). Toutefois, la tête 12 peut tout aussi bien être fixée par clipsage ou encore être réalisée de matière avec le corps 11. L'extrémité 14 de la pièce à main 1 est formée par une pièce de fond montée par clipsage ou, selon une variante de réalisation non représentée, par vissage sur un raccord 17 du corps 11.

Le corps 11 de la pièce à main 1 est conformé pour recevoir, dans le logement 111 qui est symétrique en rotation autour d'un axe 112, un support cylindrique 3 et un contenant 4 de produit pharmaceutique, le produit pharmaceutique étant une solution injectable. Le contenant 4 comprend un corps cylindrique de rotation 41 (voir figure 2) avec un axe 42 et un piston 43 pour pouvoir agir sur le contenu. En état d'utilisation, le contenant 4 est emmanché dans le support cylindrique 3, comme représenté sur la figure 2.

Le support cylindrique 3 est un élément allongé creux avec un goulot pourvu d'une partie filetée 32 à l'une de ses deux extrémités opposées et avec une ouverture axiale 36 pour l'emmanchement du contenant 4 à l'autre extrémité. Le support 3 est formé de façon que le goulot soit logé dans le passage axial 13 de la tête 12 de la pièce à main 1 et dépasse de celle-ci vers l'extérieur par la partie filetée 32 du goulot afin de pouvoir recevoir une aiguille perforante 5 par vissage. L'aiguille 5 comporte à cet effet, outre une canule 51, un embout de fixation fileté 52.

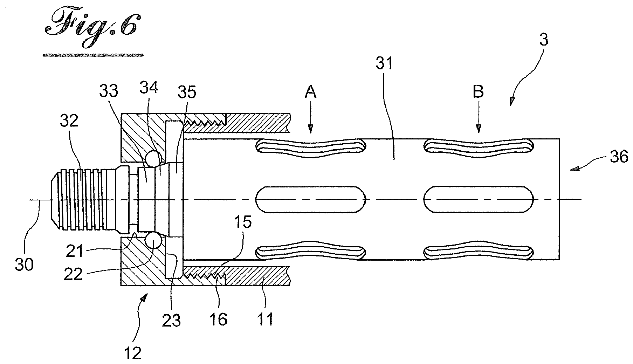

La pièce de fond 14 retient le support cylindrique 3 à l'intérieur du corps allongé 11 et le maintient en appui sur une partie intérieure de la tête 12 conformée à cet effet, comme décrit plus loin en référence à la figure 6.

En outre, la pièce de fond 14 comprend un organe 18 conformé pour être en appui sur un piston 43 du contenant 4 afin de pouvoir exercer une pression sur le produit pharmaceutique contenu dans le contenant 4. L'organe 18 est configuré pour pouvoir non seulement injecter une dose prévue du produit pharmaceutique dans le corps humain ou animal mais aussi pour introduire et maintenir sous pression, en une ou plusieurs fois, le produit pharmaceutique dans l'aiguille 5 selon des critères de fonctionnement décrits plus loin dans la présente description. Dans la pièce de fond 14 sont également logés des moyens d'entraînement 19 permettant de mettre l'ensemble support/contenant en rotation autour de l'axe longitudinal 42 du corps 41 du contenant 4.

Les figures 2 et 3 représentent respectivement en état assemblé et en état séparé le support cylindrique 3 et le contenant de produit pharmaceutique 4. Le support cylindrique 3 comporte un corps allongé creux 31 réalisé en un matériau plastique rigide. Le corps tubulaire 31 est fermé à une de ses deux extrémités opposées par le goulot fileté mentionné déjà plus haut et qui est conformé pour porter l'aiguille perforante 5. Le goulot est muni d'une lumière par laquelle l'aiguille 5 pénètre d'abord à l'intérieur du corps 31 et ensuite par le scellage élastique du contenant 4 à l'intérieur de ce dernier. Ainsi, en appuyant sur le piston 43, le produit pharmaceutique sort du contenant 4 par l'aiguille 5 et est injecté dans un corps humain ou animal. Le goulot est également pourvu d'une piste de roulement circonférentielle 33 destinée à, et conformée pour, coopérer avec un roulement mécanique 2 disposé dans la tête 12 de la pièce à main 1, comme décrit ci-après.

En effet, pour éviter l'obstruction de l'aiguille 5 par des débris produits au cours d'une phase de perforation, l'invention propose d'injecter un liquide, pendant la phase de perforation, pour lubrifier et remplir la lumière de l'aiguille. Le plus simple est alors d'utiliser pour cela le produit pharmaceutique. Ce fonctionnement empêche les débris du tissu perforé de pénétrer dans la lumière de l'aiguille.

Mais pour que ce système fonctionne, il faut réunir au moins deux paramètres :
- l'injection ne doit pas influer sur la vitesse de rotation de l'aiguille,
- le liquide doit être injecté avec une vitesse et une pression optimale.

Ces paramètres peuvent être réunis avec des moyens simples en contrôlant les paramètres d'injection (telle la vitesse, la pression et la résistance rencontrées) à l'aide d'un pousse-seringue électronique 18 et en plaçant un roulement mécanique 2, par exemple un roulement à billes oblique, à l'endroit où l'ensemble support 3/contenant 4 est en appui rotatif sur la tête 12. De cette manière, la vitesse de rotation de l'aiguille ne diminue pas lorsque l'organe 18 appuie axialement sur le piston 43, et ainsi sur l'ensemble support3/contenant 4, lorsqu'une injection de rinçage a lieu. Ainsi, l'utilisation simultanée d'une presse liquidienne à l'intérieur de l'aiguille (gérée par un pousse-seringue électronique) et le maintien de la vitesse de rotation optimale grâce à un montage sur roulement permet de supprimer l'obstruction de l'aiguille lorsque celle-ci perfore des tissus denses.

La figure 4 représente, en une vue en perspective, l'intérieur de la tête 12 de la pièce à main 1. Elle donne une vue sur le filetage intérieur 16 de la tête 12 qui coopère avec le filetage extérieur 15 du corps 11 lorsque la tête 12 est fixée sur le corps 11, et montre également le passage axial 13 et le roulement mécanique 2 qui entoure le passage axial 13. Le roulement mécanique 2 présente une surface intérieure 21 de laquelle dépassent des billes 22, et une surface transversale 23 rejoignant le filetage 16.

La figure 5 représente le support cylindrique 3 du côté du goulot en une vue en perspective avec une vue sur les faces coopérant avec le roulement mécanique 2. Le goulot comprend, partant de la partie filetée 32 vers le corps 31 du support 3, une partie cylindrique droite 33 et une partie tronconique 34 formant ensemble une piste pour les billes 22 du roulement mécanique 2. Cette piste est axialement espacée du corps 31 par une surface annulaire cylindrique 35 dont la longueur axiale détermine la distance axiale entre le corps 31 du support 3 et la surface transversale 24 de la tête 12.

Comme cela est visible sur la figure 6, les dispositions ci-avant concernant le roulement mécanique 2 et le goulot du support 3 coopèrent de la manière suivante dans la présente invention.

Pour assurer à la fois un positionnement concentrique du support 3 dans le passage 13 de la tête 12 et un espace axial entre le corps 31 du support 3 et la tête 12, le roulement mécanique 2 doit constituer à la fois un support radial et un support axial. Ceci est obtenu par une disposition adaptée des billes 22 et de la surface 21 tel que, lorsque le support 3 est logé dans la pièce à main 1 avec un contenant 4, le goulot du support 3, et plus précisément la piste de roulement formée par les parties cylindrique droite 33 et tronconique 34, est en appui sur les billes 22 sans qu'une autre partie du support 3 ne vienne en contact avec la tête 12. Les billes 22 exercent un effort suivant une résultante formant un angle de contact compris de préférence entre 10° et 30° avec l'axe 112 du corps 11 de la pièce à main 1.

De cette manière, le goulot du support 3 est maintenu par les billes en appui sur une surface annulaire concentrique droite, ici la surface 33 du goulot. En même temps, le support 3 ne peut pas venir en contact avec la surface transversale 24 de la tête 12, ce qui évite un ralentissement de la rotation du support 3 au moment d'une injection de produit pharmaceutique.

Selon l'exemple de réalisation représenté, le roulement 2 est un roulement à billes oblique. Toutefois, sans sortir du principe de la présente invention, d'autres types de roulements mécaniques peuvent également être utilisés, par exemple des roulements mécaniques contenant des rouleaux cylindriques ou coniques coopérant avec une piste en biseau formée sur le goulot à la place des surfaces 33, 34. Il est également concevable que, pour coopérer avec les billes 22, les surfaces 33, 34 soient remplacées par une surface annulaire galbée.

## Revendications

1. Instrument de chirurgie pour perforer un tissu dense d'un corps humain ou animal et pour injecter ensuite un produit pharmaceutique dans une zone du corps accessible à travers le tissu dense, l'instrument comprenant une pièce à main (1) ayant un corps allongé (11) avec un logement (111) pour loger un support cylindrique (3) libre en rotation autour d'un axe longitudinal (30) du support cylindrique conformé pour recevoir de manière échangeable et solidaire en rotation avec celui-ci, un contenant (4) de produit pharmaceutique, le corps (11) ayant deux extrémités (12, 14) opposées dont l'une (12) est pourvue d'un passage axial (13) débouchant dans le logement (111) et qui est adapté pour le passage d'une aiguille perforante (5) fixée sur une (32) des deux extrémités opposées du support cylindrique (3) et destinée à être introduite dans le contenant (4), l'autre extrémité (14) du corps (11) comprenant des moyens d'entraînement en rotation (19) du contenant (4) et de l'aiguille (5) par l'intermédiaire du support cylindrique (3),
**caractérisé en ce qu'**il comprend un organe (18) adapté pour pouvoir exercer, pendant la perforation, de façon commandée une pression sur au moins une quantité prédéterminée du produit pharmaceutique présent dans le contenant (4) inséré dans le logement (111), afin d'empêcher l'entrée de débris de perforation dans la lumière de l'aiguille (5), et **en ce que** le passage axial (13) est pourvu d'un roulement mécanique coaxial (2) constituant à la fois un support radial et un support axial servant d'appui pour l'extrémité du support cylindrique (3) conformé pour la fixation de l'aiguille d'injection perforante (5).

2. Instrument de chirurgie selon la revendication 1, **caractérisé en ce qu'**il comprend un module de commande électronique auquel l'organe de pression (18) est relié et qui est adapté pour pouvoir contrôler, pendant la perforation, la pression exercée sur au moins une quantité prédéterminée du produit pharmaceutique afin d'empêcher l'entrée de débris de perforation dans la lumière de l'aiguille (5).

3. Instrument selon l'une des revendications 1 ou 2, **caractérisé en ce que** l'extrémité de passage est en forme d'un élément distinct monté de manière amovible sur le corps (11).

4. Instrument selon l'une quelconque des revendications 1 à 3, **caractérisé en ce que** l'extrémité (32) du support cylindrique (3) conformée pour la fixation de l'aiguille d'injection perforante (5) est pourvue d'une piste de roulement circonférentielle (33) destinée à coopérer avec le roulement mécanique oblique (2) .

5. Instrument selon l'une des revendications 1 à 4, **caractérisé en ce que** la piste de roulement circonférentielle (33) est formée par un élément annulaire oblique.

6. Instrument selon l'une des revendications 1 à 5, **caractérisé en ce que** le roulement mécanique est un roulement à billes oblique (2).

7. Instrument selon l'une des revendications 1 à 5, **caractérisé en ce que** le roulement mécanique est un roulement à rouleaux cylindriques ou coniques.

8. Instrument selon l'une des revendications 1 à 7, **caractérisé en ce que** l'organe (18) agissant sur le contenant (4) est adapté pour introduire, pendant la perforation du corps humain ou animal, dans l'aiguille perforante (5) des quantités de produit pharmaceutique prédéterminées en fonction de l'avancement de la perforation.

9. Instrument selon l'une des revendications 1 à 8, **caractérisé en ce qu'**il comprend un module de commande électronique (19) relié à l'organe (18) agissant sur le contenant (4) et ayant une mémoire pour stocker des consignes d'injection de quantités prédéterminées du produit pharmaceutique en fonction de l'avancement de la perforation.

## Patentansprüche

1. Chirurgisches Instrument zum Durchstoßen eines dichten Gewebes eines Körpers eines Menschen oder Tiers und zum darauffolgenden Injizieren eines pharmazeutischen Produkts in einen Bereich des Körpers, der durch das dichte Gewebe erreichbar ist, wobei das Instrument ein Handstück (1) mit einem länglichen Körper (11) mit einer Aufnahme (111) zum Aufnehmen eines um eine Längsachse (30) des zylindrischen Trägers frei drehenden zylindrischen Trägers (3) umfasst, der ausgebildet ist, um einen Behälter (4) eines pharmazeutischen Produkts austauschbar und mit diesem rotierend fest verbunden aufzunehmen, wobei der Körper (11) zwei gegenüberliegende Enden (12, 14) hat, von denen das eine (12) mit einem axialen Durchgang (13) versehen ist, der in die Aufnahme (111) ausmündet und der für den Durchgang einer durchstoßenden Nadel (5) geeignet ist, die auf einem (32) der zwei gegenüberliegenden Enden des zylindrischen Trägers (3) befestigt ist und bestimmt, in den Behälter (4) eingeführt zu sein, wobei das andere Ende (14) des Körpers (11) Rotationsantriebsmittel (19) des Behälters (4) und der Nadel (5) mittels des zylindrischen Trägers (3) umfasst,
**dadurch gekennzeichnet, dass** es ein Organ (18) umfasst, das geeignet ist, während des Durchstoßens gesteuert einen Druck auf mindestens eine vorher festgelegte Menge des pharmazeutischen Produkts ausüben zu können, das in dem in der Aufnahme (111) eingesetzten Behälter (4) vorhanden ist, um den Eintritt von Durchstoßungstrümmern in die Öffnung der Nadel (5) zu verhindern, und dass der axiale Durchgang (13) mit einem koaxialen mechanischen Lager (2) ausgestattet ist, das gleichzeitig einen radialen Träger und einen axialen Träger bildet, die als Abstützung für das Ende des zylindrischen Trägers (3) dienen, der für die Befestigung der durchstoßenden Injektionsnadel (5) ausgebildet ist.

2. Chirurgisches Instrument nach Anspruch 1, **dadurch gekennzeichnet, dass** es ein elektronisches Steuermodul umfasst, mit dem das Druckorgan (18) verbunden ist und das geeignet ist, während des Durchstoßens den Druck steuern zu können, der auf mindestens eine vorher festgelegte Menge des pharmazeutischen Produkts ausgeübt wird, um den Eintritt von Durchstoßungstrümmern in die Öffnung der Nadel (5) zu verhindern.

3. Instrument nach einem der Ansprüche 1 oder 2, **dadurch gekennzeichnet, dass** das Durchgangsende in Form eines unterschiedlichen Elements ist, das lösbar auf dem Körper (11) angebracht ist.

4. Instrument nach einem der Ansprüche 1 bis 3, **dadurch gekennzeichnet, dass** das Ende (32) des zylindrischen Trägers (3), das für die Befestigung der durchstoßenden Injektionsnadel (5) ausgebildet ist, mit einem Umfangsrollweg (33) versehen ist, der zum Zusammenwirken mit dem mechanischen Schräglager (2) bestimmt ist.

5. Instrument nach einem der Ansprüche 1 bis 4, **dadurch gekennzeichnet, dass** der Umfangsrollweg (33) von einem schrägen Ringelement gebildet ist.

6. Instrument nach einem der Ansprüche 1 bis 5, **dadurch gekennzeichnet, dass** das mechanische Lager ein Kugel-Schräglager (2) ist.

7. Instrument nach einem der Ansprüche 1 bis 5, **dadurch gekennzeichnet, dass** das mechanische Lager ein Lager mit zylindrischen oder konischen Rollen ist.

8. Instrument nach einem der Ansprüche 1 bis 7, **dadurch gekennzeichnet, dass** das auf den Behälter (4) wirkende Organ (18) geeignet ist, während des Durchstoßens des Körpers eines Menschen oder eines Tiers in die durchstoßende Nadel (5) in Abhängigkeit von Fortschritt des Durchstoßens vorher festgelegte Mengen pharmazeutischen Produkts einzuleiten.

9. Instrument nach einem der Ansprüche 1 bis 8, **dadurch gekennzeichnet, dass** es ein elektronisches Steuermodul (19) umfasst, das mit dem auf den Behälter (4) wirkenden Organ (18) verbunden ist und einen Speicher hat, um Injektionsanweisungen in Abhängigkeit vom Fortschritt des Durchstoßens vorher festgelegter Mengen des pharmazeutischen Produkts zu speichern.

## Claims

1. A surgical instrument for perforating a dense tissue of a human or animal body and for next injecting a pharmaceutical product into a zone of the body accessible through the dense tissue, the instrument comprising a handheld part (1) having an elongate body (11) with a housing (111) for housing a cylindrical support (3) freely rotating around a longitudinal axis (30) of the cylindrical support configured to receive, exchangeably and secured in rotation therewith, a container (4) of pharmaceutical product, the body (11) having two opposite ends (12, 14), one of which (12) is provided with an axial passage (13) emerging in the housing (111) and which is suitable for the passage of a perforating needle (5) fastened on one (32) of the two opposite ends of the cylindrical support (3) and intended to be introduced into the container (4), the other end (14) of the body (11) comprising means (19) for driving the rotation of the container (4) and the needle (5) via the cylindrical support (3),
**characterized in that** it comprises a member (18) suitable for being able to exert, during the perforation, in a controlled manner, pressure on at least a predetermined quantity of the pharmaceutical product present in the container (4) inserted into the housing (111), so as to prevent perforation debris from entering the aperture of the needle (5), and **in that** the axial passage (13) is provided with a coaxial mechanical rolling bearing (2) making up both a radial support and an axial support serving as bearing for the end of the cylindrical support (3) configured to fasten the perforating injection needle (5).

2. The surgical instrument according to claim 1, **characterized in that** it comprises an electronic command module to which the pressure member (18) is connected and which is suitable for being able to control, during the perforation, the pressure exerted on at least a predetermined quantity of the pharmaceutical product in order to prevent perforation debris from entering the aperture of the needle (5).

3. The instrument according to one of claims 1 or 2, **characterized in that** the passage end is in the form of a separate element mounted removably on the body (11).

4. The instrument according to any one of claims 1 to 3, **characterized in that** the end (32) of the cylindrical support (3) configured for the fastening of the perforating injection needle (5) is provided with a circumferential rolling track (33) intended to cooperate with the oblique mechanical rolling bearing (2).

5. The instrument according to one of claims 1 to 4, **characterized in that** the circumferential rolling track (33) is formed by an oblique annular element.

6. The instrument according to one of claims 1 to 5, **characterized in that** the mechanical rolling bearing is an oblique ball bearing (2).

7. The instrument according to one of claims 1 to 5, **characterized in that** the mechanical rolling bearing is a rolling bearing with cylindrical or conical rollers.

8. The instrument according to one of claims 1 to 7, **characterized in that** the member (18) acting on the container (4) is suitable for introducing, during the perforation of the human or animal body, into the perforating needle (5), predetermined quantities of pharmaceutical product as a function of the advance of the perforation.

9. The instrument according to one of claims 1 to 8, **characterized in that** it comprises an electronic command module (19) connected to the member (18) acting on the container (4) and having a memory to store injection setpoints for predetermined quantities of the pharmaceutical product as a function of the advance of the perforation.
